# EUROPEAN PATENT SPECIFICATION

(11) **EP 2 679 242 B1**
(45) Date of publication and mention of the grant of the patent: **09.09.2020**
(21) Application number: 12750249.0
(22) Date of filing: 24.02.2012
(51) Int. Cl.: A61K 39/39, A61K 9/02, A61K 9/06, A61K 9/14, A61K 9/70, A61K 39/00

(54) **ADJUVANT FOR TRANSDERMAL OR TRANSMUCOSAL ADMINISTRATION AND PHARMACEUTICAL PREPARATION CONTAINING SAME**
HILFSSTOFF FÜR TRANSDERMALE ODER TRANSMUKOSALE VERABREICHUNG UND PHARMAZEUTISCHES PRÄPARAT DAMIT
ADJUVANT POUR L'ADMINISTRATION TRANSDERMIQUE OU TRANSMUQUEUSE ET PRÉPARATION PHARMACEUTIQUE LE CONTENANT

(30) Priority: 25.02.2011 JP 2011040283
(43) Date of publication of application: 01.01.2014
(73) Proprietor: Hisamitsu Pharmaceutical Co., Inc., Tosu-shi Saga 841-0017 (JP)
(72) Inventor: MORIMOTO, Kumi, Tsukuba-shi Ibaraki 305-0856 (JP); TOKUMOTO, Seiji, Tsukuba-shi Ibaraki 305-0856 (JP)
(74) Representative: Dr. Gassner & Partner mbB
(86) International application number: PCT/JP2012/054544
(87) International publication number: WO 2012/115222

(56) References cited:
- EP-A1- 2 123 296
- WO-A1-93/25168
- WO-A1-2007/015441
- WO-A1-2008/093772
- WO-A1-2010/001671
- WO-A1-2010/013601
- WO-A1-2010/143689
- WO-A1-2011/105508
- WO-A1-2012/115208
- WO-A2-2010/125470
- ACARTURK FUSUN ET AL: "Investigation of the effect of different adjuvants on felodipine release kinetics from sustained release monolithic films", INTERNATIONAL JOURNAL OF PHARMACEUTICS (AMSTERDAM), vol. 131, no. 2, 1996, pages 183-189, XP055251204, ISSN: 0378-5173
- AUNGST B J ET AL: "Comparison of the effects of various transmucosal absorption promoters on buccal insulin delivery", INTERNATIONAL JOURNAL OF PHARMACEUTICS, ELSEVIER BV, NL, vol. 53, no. 3, 1 August 1989 (1989-08-01) , pages 227-235, XP025543981, ISSN: 0378-5173, DOI: 10.1016/0378-5173(89)90316-5 [retrieved on 1989-08-01]
- GHUMMAN B. ET AL.: 'Chemical chaperones enhance superantigen and conventional antigen presentation by HLA-DM-deficient as well as HLA-DM-sufficient antigen-presenting cells and enhance IgG2a production in vivo.' JOURNAL OF IMMUNOLOGY vol. 161, no. 7, 1998, pages 3262 - 3270, XP055120048
- STOJANOVIC M. ET AL.: 'The context of tetanus toxoid application influences the outcome of antigen-specific and self-directed humoral immune response.' MICROBIOLOGY AND IMMUNOLOGY vol. 53, no. 2, 2009, pages 89 - 100, XP055120052
- WANG J. ET AL.: 'Effect of polyethylene glycol as adjuvant on hepatitis B virus DNA vaccine in vitro.' ACTA MICROBIOLOGICA SINICA (WEI SHENG WU XUE BAO) vol. 50, no. 7, 2010, pages 949 - 954, XP008170109
- FERRO V.A. ET AL.: 'Immune responses to a GnRH-based anti-fertility immunogen, induced by different adjuvants and subsequent effect on vaccine efficacy.' VACCINE vol. 22, no. 8, 2004, pages 1024 - 1031, XP004489113

## Description

### [Technical Field]

The present invention relates to an adjuvant mainly for transdermal or transmucosal administration, used for safe and efficient enhancement of immune activity of the skin.

### [Background Art]

The skin consists of the stratum corneum that is the outermost layer, the epidermis, the cutis and the subcutaneous connective tissue; usually, the stratum corneum that consists of a layer of dead cells and lipid bilayers shows a strong barrier function for many substances.

In the epidermis layer, antigen-presenting cells called Langerhans cells are present, serving immune functions. The mucous membrane is also a boundary with external environments, covering the oral cavity, nasal cavity, respiratory organs, digestive organs and genital organs, and it has the same structure as the skin except that there is no stratum corneum, i.e., the outermost layer of the skin. The mucous membrane is in contact with various foreign bodies through food intake, breathing, etc., and for example, it is a main passage for pathogenic microorganisms to enter into the body of a host. Therefore, the immunological defense mechanism in the mucous membrane is also important as a life barrier.

Langerhans cells capture a protein antigen that has entered the skin, disintegrate it internally and express a peptide fragment on an MHC molecule. The MHC-peptide complex moves from afferent lymph vessel to the subcortical layer of the regional lymph node, and comes into contact via T cells and interdigitating cells. Due to such movement of the Langerhans cells, the antigen is efficiently conveyed from the skin to helper T cells (TH cells) present in the lymph node. Langerhans cells have an abundance of MHC class II molecules necessary for presenting antigen to TH cells.

In recent years vaccines are shifting from live vaccines to inactivated vaccines (whole particles, components, vaccines, etc.) for the purpose of improving safety; while the risk of infection is reduced, addition of an adjuvant is required in many cases in order to compensate for the immune effects. An adjuvant is a substance that enhances immune responses to antigens, and in vaccination, it is very useful for reducing the dose and administration frequency of vaccines, and for accelerating the rise of immune response.

Many studies on adjuvants have been carried out to date, and as some examples, aluminum salts, immune-stimulating complexes (ISCOMs), substances derived from bacteria, etc. are known. However, many of these adjuvants are often directly administered subcutaneously or intramuscularly, and in such cases, tissue damage such as contact hypersensitivity, subcutaneous nodule and granuloma is induced. Therefore, in the immunostimulation such as human vaccination, there is a high demand for adjuvants and preparations that can be administered safely and effectively.

As adjuvants, various vaccine formulations comprising an attenuated pathogen or a protein subunit antigen have been developed extensively. In most cases, conventional vaccine preparations comprise an adjuvant that enhances immune responses. For example, an adjuvant that forms a depot is well known. This adjuvant makes the antigen administered be absorbed or settle, and forms a depot at the site of injection. As typical depot-forming adjuvants, aluminum compounds such as aluminum phosphate and aluminum hydroxide gel, and oil-in-water emulsions etc. are mentioned.

However, while the depot-forming adjuvants enhance antigenicity, they have a problem in the use because they bring about local tissue damage such as erythema, contact hypersensitivity and granuloma formation when administered subcutaneously or intramuscularly. Furthermore, a problem of absorbability of aluminum salt also occurs in the transdermal administration. Such a problem of transdermal absorbability of adjuvants themselves also occurs in immune-stimulating complexes (ISCOMs), substances derived from bacteria and cytokines, used as an adjuvant. For example, muramyl dipeptide is known to cause, at the time of injection, a pyrogenic response that is an influenza-like symptom, or Reiter's syndrome, general arthralgia, and further, in some cases anterior uveitis, arthritis and urethritis.

As described above, conventional adjuvants have often caused severe local tissue damage at the time of subcutaneous administration or intramuscular administration. Therefore, in order to avoid this local tissue damage, transdermal administration was considered, but the conventional adjuvants are macromolecules such as an immune-stimulating complex (ISCOM) and a substance derived from bacteria, or aluminum compounds, etc., all of which are compounds that are not suitable for transdermal administration.

In addition, recently, external dosage forms by iontophoresis or a device equipped with microneedles as means for increasing the permeation have been studied, but at present, when the adjuvant as well as the macromolecular antigen is poorly absorbable, it is impossible that the antigen and adjuvant are permeated efficiently.

For example, Patent Publication 1 discloses electroporation as a method for delivery of macromolecular antigens into the epidermal cells, but it does not disclose adjuvants.

Patent Publication 2 discloses a skin patch having a microprojection array, a reservoir containing an antigenic agent and an immune response enhancing adjuvant, and a method for use thereof to vaccinate animals (for example, humans). However, the adjuvants described in said Patent Publication are only metal salts and macromolecules (peptide, etc.), and there is no description of adjuvants having skin permeability.

Patent Publication 3 discloses long-chain aliphatic alcohols, esters thereof with C1 to C6 alkanoic acids, or certain esters of long-chain fatty acids with alkanols and polyols, as a low molecular adjuvant administered by infusion. Furthermore, Patent Publication 4 describes that an adjuvant which is a hydroxyl unsaturated fatty acid or its derivative is orally administered. However, its immune response against an antigen in the transdermal administration, in particular an increase in the antibody titer, has not been specifically described.

Furthermore, in Patent Document 5, a method for local administration consisting of a step of administering a mixture of an antigen and a lipophilic solvent, followed by a step of administering an inducer of migration of Langerhans cells is disclosed. However, according to the description of this document, substances that promote the induction of Langerhans cells are limited to divalent unsaturated carboxylate esters, such as dibutyl phthalate.

Patent Publication 6 discloses a dry preparation comprising a cholera toxin or a related ADP-ribosylating toxin as an adjuvant. In such a preparation, it is speculated that the cholera toxin or related ADP-ribosylating toxin as an adjuvant penetrates the skin, and induces an immune response. Meanwhile, there is a little information about safety of such an adjuvant, and there are disadvantages of high cost and low skin permeability because it is a polymer.

Patent Publication 7 discloses a method of delivering an immunogenic composition to the intradermal compartment, and an excipient used in this method. However, there is no description of a polyhydric alcohol as an immunostimulatory adjuvant to be administered separately from the antigen, as well as an immunostimulating method using thereof.

Patent Publications 8 and 9 disclose aliphatic adjuvants, free fatty acids and fatty acid derivatives as an adjuvant for transdermal or transmucosal administration. However, adjuvant effects of polyhydric alcohols such as glycerin and their derivatives are not disclosed.

### [Citation List]

### [Patent Literature]

[Patent Publication 1] JP, A, 2002-535100
[Patent Publication 2] JP, A, 2004-538048
[Patent Publication 3] JP, A, 2004-526757
[Patent Publication 4] WO 2002/017691
[Patent Publication 5] JP, A, 2002-512186
[Patent Publication 6] JP, A, 2001-517233
[Patent Publication 7] JP, A, 2007-516968
[Patent Publication 8] WO 2002/015441
[Patent Publication 9] WO 2008/093772

### [Summary of Invention]

### [Technical Problems]

As described above, conventional adjuvants used for injection, etc. have a problem of local tissue damage and others. In addition, while percutaneous absorption preparations are characterized by easiness and high safety compared to injections, there are a very few number of substances, in particular low molecular compounds, that efficiently exhibit an action of adjuvant by percutaneous administration. Furthermore, an adjuvant that can be provided inexpensively and safely is strongly desired at clinical sites as well.

It is therefore an object of the present invention to provide a low molecular adjuvant, that can be administered safely without inducing skin irritation etc. by transdermal or transmucosal administration and that is for the purpose of efficiently enhancing immunogenicity of antigens.

### [Solution to Problem]

While carrying out intensive investigations in order to solve the above-mentioned problems, the present inventors have found that, certain low molecular compounds, in particular polyhydric alcohols such as glycerin as well as derivative thereof, not only show a strong immunoenhancing action, but also avoid skin irritation and tissue damage in transdermal or transmucosal administration; and as a result of further research, the present invention has been accomplished.

Namely, the present invention provides the following [1] to [12].
[1] An adjuvant comprising one or more selected from the group consisting of glycerin and propylene glycol that are polyhydric alcohols for use in the enhancement of immune activity when administered by transdermal or transmucosal application after antigen administration, wherein the adjuvant is contained at 85-100 wt% in a pharmaceutical preparation, wherein the use comprises administration of the antigen by subcutaneous injection or intradermal injection, transdermal or transmucosal administration of the antigen or administration of the antigen with microneedles before the adjuvant is applied.
[2] The adjuvant according to [1] for use according to [1], wherein the pharmaceutical preparation is an ointment, a cream, a gel, a suppository, a hydrogel patch, a lotion, a solution, an impregnated-type preparation, or a blister.
[3] The adjuvant according to [1] or [2] for use according to [1], wherein the adjuvant is contained at 95-100 wt% in the pharmaceutical preparation.
[4] The adjuvant according to any one of [1] to [3] for use according to [1], wherein the pharmaceutical preparation is a matrix-type or laminated-type tape preparation or a reservoir-type preparation.
[5] The adjuvant according to any one of [1] to [4] for use according to [1], which use comprises application of the pharmaceutical preparation to the intact skin or mucous membrane, or the skin or mucous membrane that had been subjected to physical or chemical treatment.
[6] The adjuvant according to [5] for use according to [1], wherein the physical or chemical treatment is at least one of laser irradiation, skin abrading, or microneedle treatment, thermal treatment, ultrasonic treatment, electric field treatment, magnetic field treatment, pressure treatment or alkali treatment.
[7] The adjuvant according to any one of [1] to [6] for use according to [1], which use comprises application of the pharmaceutical preparation by at least one of skin abrading, microneedles and needle-free injection.
[8] The adjuvant according to any one of [1] to [7] for use according to [1], wherein a part or the entire surface of the needle part of the microneedles is coated with an antigen and/or an adjuvant.
[9] The adjuvant according to any one of [1] to [8] for use according to [1], which use comprises application of the pharmaceutical preparation by at least one of lamellar structural changes, hydration, degeneration, small hole formation, peeling, or bypass formation in the stratum corneum.
[10] The adjuvant according to [9] for use according to [1], which use comprises application of the pharmaceutical preparation by at least one of iontophoresis, sonophoresis, or electroporation.
[11] The adjuvant according to any one of [1] to [10] for use according to [1], wherein the pharmaceutical preparation is comprised in a kit.
[12] The adjuvant according to [11] for use according to [1], wherein the kit comprises an antigen or a vaccine, and an apparatus for antigen administration.

### [Advantageous Effects of Invention]

Glycerin and its derivatives have a good record as medicines, have been used as injections as well, and they are inexpensive and highly safe. To date, it has been thought that in transdermal or transmucosal administration, adjuvant effect can be obtained only by aliphatic alcohols and fatty acids having high transdermal absorbability as described in Patent Documents 8 and 9; however, the present inventors showed that adjuvant effect in transdermal or transmucosal administration can be exerted by the polyhydric alcohols of the present invention.

As described above, according to the present invention, a safe adjuvant having a strong immunoenhancing action in transdermal or transmucosal administration, without causing skin irritation and tissue damage, is provided. In addition, unlike adjuvants as permeation or absorption accelerators, the adjuvant of the present invention exerts excellent immunoenhancing effect by being applied with a high concentration to the skin. Therefore, the adjuvant of the present invention is particularly excellent as an adjuvant with less skin irritation for transdermal or transmucosal administration.

In addition, unlike adjuvants as permeation or absorption accelerators, the adjuvant of the present invention may not be mixed with an antigen for administration, and the present adjuvant can be administered via a route different from the route of the antigen. In particular the adjuvant of the present invention can provide excellent adjuvant effect by its transdermal or transmucosal administration independent from antigens and vaccines. Accordingly, upon administration of the adjuvant, it is not necessary to consider the dose and other conditions of antigens, etc., and the concentration, application time, and dose of the adjuvant itself can be arbitrary selected. Furthermore, swelling of the administration site occurred at the time of administration of antigens and the pain associated with it can be avoided.

In addition, when an adjuvant is mixed with an antigen, etc. for administration, it is necessary to consider a possibility that immunogenicity against other physiologically active substances and formulations increases, and that sensitization against them occurs. Therefore, we can say that usefulness and safety is extremely high with the present invention, as compared to the case of administration by mixing the adjuvant.

Furthermore, the adjuvant for transdermal or transmucosal administration of the present invention has a low melting point and a low molecular weight, and therefore shows high transdermal or transmucosal absorbability, so that application to preparations for various kinds of transdermal absorption preparations, for example, solution, patches, ointment, gels, impregnated-type preparation, creams, lotion, etc. can be realized, and they can be provided at a low cost.

### [Brief Description of Drawings]

[Fig. 1] Figure 1 shows effects of the adjuvant of the present invention to increase IgG antibody titer.
[Fig. 2] Figure 2 shows adjuvant effects of the adjuvant (glycerin) of the present invention.
[Fig. 3] Figure 3 shows adjuvant effects of the present invention by application with intradermal injection of OVA antigen and by application with microneedles.
[Fig. 4] Figure 4 shows time-course changes in IgG antibody titer by application of the adjuvant of the present invention with intradermal injection of OVA antigen and with microneedles.
[Fig. 5] Figure 5 shows adjuvant effects of the adjuvant (glycerin and propylene glycol (PG)) of the present invention as well as of macrogol, monoglyceryl oleate (GMO) and triacetin.

### [Description of Embodiments]

The adjuvant of the present invention comprises one or more substances selected from the group consisting of glycerin and propylene glycol that are polyhydric alcohols.

The adjuvant of the present invention may be used alone, or in a combination thereof. In particular, when a synergistic effect between adjuvants is present, these adjuvants are preferably used in a combination. In other cases, an adjuvant is used alone, or it may be used in a combination depending on an objective.

The adjuvant of the present invention can easily increase antigen effect particularly by its transdermal of transmucosal administration, and the adjuvant itself possesses an effect to increase the antigen effect. Accordingly, the adjuvant can be administered by a dosage form different from that of antigens and vaccines, or via an independent administration route.

Furthermore, the adjuvant of the present invention can be administered at a time point and in procedure different from those for antigens, etc.; the adjuvant of the present invention can be administered in an identical or different dosage form prior to the antigen administration, simultaneously with the antigen administration, or after the antigen administration. Accordingly, without selecting the dosage form of antigens etc., the present adjuvant can be applied to an appropriate site of a subject at an appropriate time point.

The adjuvant of the present invention is contained in a pharmaceutical preparation, and is used at 85-100 wt% in the pharmaceutical preparation, preferably 95-100 wt%.

By means of using a high concentration of the adjuvant of the present invention, a difference in the effect between the present adjuvant and other components used in the pharmaceutical preparation, such as an excipient, becomes clear.

By comprising the adjuvant of the present invention in a conventionally-used transdermal administration preparation, the adjuvant can be non-invasively administered into the body in a form of externally-applied pharmaceutical preparation. As an example of such form of pharmaceutical preparation, a dosage form containing an adjuvant of the present invention, with which transdermal administration of the adjuvant is possible, is preferred, and it can be selected from hydrogel patches, patches, ointment, creams, solution, gels, impregnated-type preparations, and lotion, etc. depending on the necessity.

An impregnated-type preparation or an impregnated-type formulation is a preparation wherein a pad is impregnated with a solution comprising an active ingredient to retain the solution and the pad is covered by an adhesive covering material. Its composition is not particularly limited, and may include a support, a backing member non-permeable to the solution (film), an adhesive covering agent, a pad, and a liner, etc.; and the preparation can be made to hold the solution, ointment or gel that has been impregnated into the pad part, in a stable manner. Furthermore, the following preparations are also included in this type: a preparation wherein a solution, etc. is stored in a blister container, etc., which is impregnated in the pad part at the time of application.

As the pad of the present invention, natural fabric materials such as gauze and absorbent cotton, etc., synthetic fiber fabric materials such as polyester, polyethylene and polyvinyl, as well as pulp can be used, and a combination thereof processed into woven fabric and non-woven fabric can be used.

The pharmaceutical preparation can be adopted in a kit for immunostimulation method. The kit is not limited as long as it comprises the pharmaceutical preparation, and it may also comprise an antigen or vaccine, or an apparatus for antigen administration. The apparatus for antigen administration may be an administration apparatus such as microneedles or injection syringe.

Preferably, the kit comprises the pharmaceutical preparation, an antigen or vaccine, or an apparatus for antigen administration. One embodiment of the kit includes, for example, a kit comprising microneedles in which the needle part is coated with an antigen, as well as a patch preparation containing the adjuvant of the present invention.

Application of the adjuvant of the present invention is carried out preferably in a transdermal or transmucosal dosage form.

It is preferable that the adjuvant of the present invention is not mixed with an antigen and the adjuvant is administered via a different route. Thus, the adjuvant of the present invention may be applied in a dosage form different from antigens and vaccines, via an independent route.

In addition, the site of application of the adjuvant of the present invention may be identical to or different from a site or region of application of antigens, etc.

Although it is not particularly limited, a preferred combination of dosage forms includes a method wherein an antigen, etc. is injected subcutaneously or administered via puncture, and an adjuvant, etc. is administered transdermally or transmucosally. In addition, when using an administration apparatus such as microneedles, it is possible to apply the adjuvant of the present invention while applying such administration apparatus.

In the present specification, the matrix-type tape preparation refers to the one, among tape preparations, having an adhesive layer in which a pharmacologically active substance is dispersed and contained in an adherent base comprising essentially a gum-like (glass-like) polymer or a gel, wherein the adhesive layer has a support on its one surface and a detachment liner on its other surface. In addition, the laminated-type tape preparation refers to the one, among tape preparations, having a plurality of adhesive layers in which a pharmacologically active substance is dispersed and contained in an adherent base, wherein the adhesive layer has a support bonded on its one surface and a detachment liner bonded on its other surface. The reservoir-type patch preparation refers to the one having a reservoir to store a pharmacologically active substance, wherein the reservoir has a backing member (a support) non-permeable for a drug on its one surface and a detachment liner or a drug-permeable adhesive layer with a detachment liner on its other surface.

In addition, such transdermal or transmucosal administration preparation can be manufactured by an ordinary method using, as the base, arbitrary ingredients such as a solubilizer, a solubilizing agent, a pH regulator, a preservative, an absorption accelerator, a stabilizing agent, a filler, a thickener, an adhesive, and a wetting agent in combination with the adjuvant of the present invention. In addition, among the pharmaceutical preparations, pharmaceutical preparations of other dosage forms can also be manufactured by an ordinary method.

For example, among the ingredients of the base in the transdermal or transmucosal administration preparation, as a thickener, the one that can stably retain 30% to 80% of moisture and has water retentivity is preferable. As the specific examples thereof, those of plant origin such as guar gum, locust bean gum, carrageenan, alginic acid, sodium alginate, agar, gum Arabic (acacia gum), tragacanth gum, karaya gum, pectin and starch, those of microbial origin such as xanthan gum, natural polymers of animal origin such as gelatin and collagen, celluloses such as methyl cellulose, ethyl cellulose, hydroxyethyl cellulose and sodium carboxymethylcellulose, semisynthetic polymers such as starch, e.g., soluble starch, carboxymethyl starch and dialdehyde starch, vinyls such as polyvinyl alcohol, polyvinyl pyrrolidone and polyvinyl methacrylate, acryls such as polyacrylic acid and sodium polyacrylate, as well as water-soluble polymers such as synthetic polymers, e.g., polyethylene oxide, and methyl vinyl ether/maleic anhydride copolymer are suitably used. In particular, sodium polyacrylate is preferable. This is because gel strength is high and water retentivity is excellent. Furthermore, sodium polyacrylate having a mean degree of polymerization of 20,000 to 70,000 is preferable. This is because, as the mean degree of polymerization becomes smaller than 20,000, the thickening effect tends to become poor and the gel strength tends to be insufficient, and as the mean degree of polymerization becomes larger than 70,000, the thickening effect tends to be excessively strong, and workability tends to decrease.

In addition, by using two or more of the above-described water-soluble polymers together, for example a polymer complex is formed with a strong ion polymer of sodium polyacrylate, and an elastic gel with far larger gel strength can be obtained.

Glycerin and propylene glycol, etc. can exhibit the effect as a wetting agent, but as necessary, polyhydric alcohol such as sorbitol may further be used as a wetting agent. Furthermore, as a filler, kaolin, zinc oxide, talc, titanium, bentonite, aluminum silicate, titanium oxide, zinc oxide, aluminum metasilicate, calcium sulfate, calcium phosphate, etc. may be added. The amount of blending of the wetting agent or filler is preferably 0.1 to 30 wt% based on the total composition of the adhesive layer, and more preferably, 0.1 to 20 wt%.

In addition, as a solubilizing agent or an absorption accelerator, propylene carbonate, crotamiton, I-menthol, peppermint oil, limonene, diisopropyl adipate, etc. may be added, and as pharmaceutical auxiliaries, methyl salicylate, glycol salicylate, I-menthol, thymol, peppermint oil, vanilylamide nonylate, red pepper extract, etc. may be added. Furthermore, a stabilizer, an antioxidant, an emulsifier, a surfactant, etc. may be added as necessary.

The surfactant may be any of non-ionic surfactants and ionic surfactants (cationic, anionic, zwitterionic); from the aspect of safety, an non-ionic surfactant that is usually used for a pharmaceutical base is desirable. More particularly, examples include sugar alcohol fatty acid esters such as sucrose fatty acid ester, sorbitan fatty acid ester, glycerol fatty acid ester, polyglycerol fatty acid ester, propylene glycol fatty acid ester, polyoxyethylene sorbitan fatty acid ester, polyoxyethylene glycerol fatty acid ester, polyethylene glycol fatty acid ester, polyoxyethylene castor oil, polyoxyethylene hydrogenated castor oil, etc.

In the transdermal administration preparation, a cross-linking agent and a polymerization agent, etc. may be added as necessary. A plaster can be made robust and water retentivity can be provided. This cross-linking agent and polymerization agent are selected appropriately in accordance with, for example, thickeners. For instance, in cases where polyacrylic acid or polyacrylate is employed as a thickener, compounds having at least two epoxy groups in a molecule, inorganic acid salts such as hydrochloride, sulfate, phosphate and carbonate of Ca, Mg, Al, etc., organic acid salts such as citrate, tartrate, gluconate and stearate, oxides such as zinc oxide and silicic anhydride, polyvalent metal compounds like hydroxides such as aluminum hydroxide and magnesium hydroxide are suitably used.

In addition, in cases where polyvinyl alcohol is employed as a thickener, adipic acid, thioglycolic acid, an epoxy compound (epichlorohydrin), aldehydes, an N-methylol compound, complexing substances such as compounds of Al, Ti, Zr, Sn, V, Cu, B, Cr, etc. are suitably used.

Furthermore, in cases where polyvinyl pyrrolidone is employed as a thickener, methyl vinyl ether/maleic anhydride copolymer, a polyacid compound or alkali metal salts thereof (polyacrylic acid and tannic acid and derivatives thereof), etc., are suitably used. In addition, in cases where polyethylene oxide is employed as a thickener, peroxides, polysulfone azide, etc. are suitably used.

Furthermore, in cases where methyl vinyl ether/maleic anhydride copolymer is employed as a thickener, polyfunctional hydroxy compound, polyamine, iodine, gelatin, polyvinyl pyrrolidone, iron, mercury, lead salt, etc. are suitably used. In cases where gelatin is employed as a thickener, aldehydes such as formaldehyde, glutaraldehyde and dialdehyde starch, diepoxides such as glyoxal and butadiene oxide, diketones such as divinyl ketone and diisocyanates are suitably used. In addition, in cases where sodium polyacrylate is employed as a thickener, it is preferable that a polyvalent metal salt such as lithium hydroxide, zinc hydroxide, aluminum hydroxide and sodium borate is added as a cross-linking agent.

In particular, zinc salt and aluminum salt are preferable. This is because cross-linking reaction is promoted. The concentration of a polyvalent metal salt added as a cross-linking agent is preferably 0.5 to 1.5 equivalents based on 1 equivalent of the thickener (or water-soluble polymer). This is because, by setting the concentration of the polyvalent metal salt to 0.5 equivalent or more, the reaction is promoted and gel strength increases; by setting the concentration of the polyvalent metal salt to 1.5 equivalent or less, the reaction is carried out in a moderate rate and gelation can be equalized, and the workability is enhanced.

As an adhesive used for the patches, an acrylic polymer or a rubber polymer is preferable. The acrylic polymer is not particularly limited as long as it is copolymerized with at least one of (meth)acrylic acid derivatives represented by 2-ethylhexyl acrylate, methyl acrylate, butyl acrylate, hydroxyethyl acrylate, 2-ethylhexyl methacrylate, etc.; preferably, the one containing 50% or more of 2-ethylhexyl acrylate is desirable. Specific adhesives that can be used include those disclosed in Japanese Pharmaceutical Excipients Directory 2000 (edited by Japan Pharmaceutical Excipients Council) as adhesives, such as acrylic acid/octyl acrylate ester copolymer, 2-ethylhexyl acrylate/vinyl pyrrolidone copolymer solution, acrylate ester/vinyl acetate copolymer, 2-ethylhexyl acrylate/2-ethylhexyl methacrylate/dodecyl methacrylate copolymer, methyl acrylate/2-ethylhexyl acrylate copolymerized resin emulsion, and acrylic polymers included in acrylic resin alkanolamine liquid, etc., DURO-TAK acrylic adhesive series (Henkel Corporation), Eudragit series (Higuchi Inc.) and others.

The rubber polymers include styrene-isoprene-styrene block copolymer (hereinafter, abbreviated as SIS), isoprene rubber, polyisobutylene (hereinafter, abbreviated as PIB), styrene-butadiene-styrene block copolymer (hereinafter, abbreviated as SBS), styrene-butadiene rubber (hereinafter, abbreviated as SBR), polysiloxane, etc., and among these, SIS, PIB and polysiloxane are preferable, and SIS and PIB are particularly preferable.

Two or more of such hydrophobic polymers may be mixed and used, and the amount of blending of these polymers based on the weight of the total composition of these polymers is preferably 5 to 90 wt%, and more preferably 10 to 70 wt%, taking into account the formation of the adhesive layer and sufficient permeability to the skin.

A plasticizer may be added to an adhesion matrix (adhesive layer) of the patches. Plasticizers that can be used include petroleum oil (for example, paraffinic process oil, naphthenic process oil, aromatic process oil, etc.), squalane, squalene, plant oil (for example, olive oil, camellia oil, castor oil, tall oil and peanut oil), silicon oil, dibasic acid ester (for example, dibutyl phthalate, dioctyl phthalate, etc.), a liquid rubber (for example, polybutene, liquid isoprene rubber), liquid fatty acid esters (isopropyl myristate, hexyl laurate, diethyl sebacate, diisopropyl sebacate), diethylene glycol, polyethylene glycol, glycol salicylate, propylene glycol, dipropylene glycol, triacetin, triethyl citrate, crotamiton, etc. Among these, liquid paraffin, liquid polybutene, isopropyl myristate, diethyl sebacate and hexyl laurate are preferable, and in particular, liquid polybutene, isopropyl myristate and liquid paraffin are preferable.

Two or more kinds of these ingredients may be mixed and used, and such a plasticizer can be added in an amount of 10 to 70 wt%, preferably 10 to 60 wt%, more preferably 10 to 50 wt%, based on the total composition of the adhesive layer, taking into account sufficient permeability to the skin and maintenance of sufficient cohesive power as the patches.

In cases where the adhesive strength is insufficient in the adhesion matrix (adhesive layer) of the patches, it is desirable to add a tackifier resin, and the tackifier resin that can be used includes a rosin derivative (for example, rosin, glycerol ester of rosin, hydrogenated rosin, glycerol ester of hydrogenated rosin, pentaerythritol ester of rosin, etc.), an alicyclic saturated hydrocarbon resin (for example, ARKON P100, Arakawa Chemical Industries), an aliphatic 1 series hydrocarbon resin (for example, Quintone B170, Zeon Corporation), a terpene resin (for example, Clearon P-125, Yasuhara Chemical) and a maleic acid resin, etc. In particular, the glycerol ester of hydrogenated rosin, the alicyclic saturated hydrocarbon resin, the aliphatic hydrocarbon resin and the terpene resin are preferable.

Such a tackifier resin can be added in an amount of 5 to 70 wt%, preferably 5 to 60 wt%, more preferably 10 to 50 wt% based on the total composition of the adhesive layer, taking into account sufficient adhesive strength as the patches and irritating property to the skin at the time of detachment.

An absorption accelerator may be added to the adhesion matrix (adhesive layer) of the patches, and the absorption accelerator that can be used may be any compound as long as its absorption promotion action on the skin is conventionally recognized; for example, a fatty acid having 6 to 20 carbon atoms, an aliphatic alcohol, a fatty acid ester, amide or ether, an aromatic organic acid, an aromatic alcohol, an aromatic organic acid ester or ether (the above ones may be either saturated or unsaturated, and further may be either cyclic, linear chain, or branched), and furthermore, lactate esters, acetate esters, monoterpene compounds, sesquiterpene compounds, azone, azone derivatives, pirotiodecane, glycerol fatty acid esters, propylene glycol fatty acid esters, sorbitan fatty acid esters (Span series), polysorbates (Tween series), polyethylene glycol fatty acid esters, polyoxyethylene hardened castor oil (HCO series), polyoxyethylene alkyl ethers, sucrose fatty acid esters, vegetable oils, etc. are mentioned.

Such an absorption accelerator may be used alone or two or more of them may be mixed and used, which can be added with preferably 0.01 to 40 wt%, more preferably 0.05 to 10 wt%, particularly preferably 0.1 to 5 wt%, based on the weight of the total composition of the adhesive layer, taking into account sufficient permeability to the skin as the patches and irritating property to the skin such as flare and edema.

Furthermore, it is preferable that, in order to obtain immunostimulation effect, the adjuvant of the present invention is administered separately from antigen. However, in accordance with the present disclosure, it may be also possible to co-administer the adjuvant with an antigen in the body. In this case, when the antigen can be administered transdermally, a transdermal non-invasive preparation comprising the adjuvant and the antigen can be prepared. As the form of such transdermal administration preparation, a transdermal administration preparation such as a hydrogel patch, a patch preparation, an ointment, creams, a solution, an impregnated-type preparation, gels, and a lotion can be selected as necessary, which is not particularly limited as long as it is in a dosage form comprising an antigen and a low molecular adjuvant, capable of being administered transdermally; an impregnated-type preparation is preferable. As described above, in the present specification, a patch preparation includes a matrix-type tape preparation, a laminated-type tape preparation and a reservoir-type patch preparation.

In addition, such transdermal preparations can also be manufactured by an ordinary method using arbitrary ingredients as a base, such as a solubilizer, a solubilizing agent, a pH regulator, a preservative, an absorption accelerator, a stabilizer, a filler, a thickener, and an adhesive, combined with an antigen and the adjuvant of the present invention. In addition, as the above-described absorption accelerator, those which can enhance the skin permeability of the adjuvant and/or antigen can be added to the base, but the adjuvant of the present invention is able to enhance the immunogenicity of antigens even without such an absorption accelerator.

Meanwhile, in cases where an antigen used concomitantly does not have a sufficient transdermal or transmucosal activity, only the adjuvant of the present invention may be administered transdermally or transmucosally, and the antigen used concomitantly may be administered non-transdermally or non-transmucosally; for example, administration via injection may be considered.

A preferable method of administration of the pharmaceutical preparation is to apply the pharmaceutical preparation comprising the adjuvant of the present invention (particularly preferably a patch preparation) before or after the antigen is administered non-transdermally or non-transmucosally, or at the same time as the antigen is administered. It is particularly preferable that the pharmaceutical preparation is applied after the antigen is administered non-transdermally or non-transmucosally; in such a case, the pharmaceutical preparation can be continuously attached while the antigen is administered. For example, the pharmaceutical preparation may be applied during the antigen is administered via microneedles, etc. In addition, in cases where the pharmaceutical preparation is attached before the antigen is administered non-transdermally or non-transmucosally, the pharmaceutical preparation may be continuously attached during administration of the antigen, and furthermore, also after administration of the antigen.

The amount of blending of the antigen and adjuvant in the above-described combination preparation of antigen and adjuvant can be determined appropriately depending on the combination of the antigen and adjuvant; although there is no particular limitation, use of an adjuvant with high concentration is preferred.

In addition, the content of the adjuvant in such a preparation is not particularly limited, and the content that can exert sufficient antigen immune response by transdermal administration is preferred, and an adjuvant with high concentration is preferred.

In the present specification, "antigen" means a substance that binds to an antigen receptor on an immune cell and causes an immune response, and examples include, without limitation, polynucleotides (DNA vaccine, RNA vaccine) and protein-based vaccines. Specific examples include antigens in the form of protein, polysaccharide, oligosaccharide, lipoprotein, attenuated or inactivated viruses such as cytomegalovirus, hepatitis B virus, hepatitis C virus, human papilloma virus, rubella virus and varicella zoster, attenuated or inactivated bacteria such as pertussis bacteria, tetanus bacillus, diphtheroid, Group A Streptococcus, *Legionella pneumophila, Neisseria meningitidis, Pseudomonas aeruginosa, Streptococcus pneumoniae, Treponema pallidum* and cholera bacillus, and mixtures thereof.

Commercially available vaccines comprising an antigenic active substance may also be used in the present invention. And further, influenza vaccine, Lyme disease vaccine, rabies vaccine, measles vaccine, epidemic parotitis vaccine, varicella vaccine, smallpox vaccine, hepatitis vaccine, pertussis vaccine and diphtheria vaccine, as well as antigens used in vaccine therapy such as the one for cancer, arteriosclerosis, nervous system disease and Alzheimer's disease are also included.

In addition, the antigen may be an allergen substance having antigenicity (sensitization properties), and various metals and chemical substances are included. For example, in the case of allergy test to clarify antigens of atopic dermatitis and its treatment, house dust such as dust and inactivated mites as well as various kinds of pollen may be used. In addition, antigens recognized by inflammatory T cells related to T cell-mediated autoimmune disease or symptoms are also included.

The administration route of these antigens includes, but is not particularly limited to, administration methods by injection (subcutaneous, intradermal), transmucosal and transdermal administration. In the case of transdermal administration, a transdermal administration means in accordance with the skin permeability of the antigen and a necessary dosage is selected.

By administering the adjuvant of the present invention with transdermal or transmucosal administration means, Langerhans cells of the skin or mucous membrane are activated, and transmitted efficiently from the skin or mucous membrane to TH cells present within a lymph node, thereby accomplishing a high immune response. By this, easy evaluation of antigenicity of external pharmaceuticals, cosmetics or allergen substances, and prevention or treatment of infectious disease, cancer and allergy, etc. by vaccines, and treatment of T cell-mediated autoimmune disease, etc. are enabled.

The preferable method of administration of the pharmaceutical preparation is a method in which the adjuvant pharmaceutical preparation of the present invention is transdermally or transmucosally administered before or after antigen administration, or simultaneously with antigen administration, and more preferably, to attach the pharmaceutical preparation comprising the adjuvant of the present invention after administration of an antigen. In addition, in cases where the pharmaceutical preparation is attached before the antigen is administered, the pharmaceutical preparation may be continuously attached during antigen administration and also after antigen administration.

The duration of attachment of the pharmaceutical preparation is not particularly limited as long as the adjuvant of the present invention can sufficiently penetrate the skin or mucous membrane to exert its effect sufficiently, even when the attachment is carried out before or after antigen administration, or even when the attachment is carried out simultaneously with the antigen administration; and the duration of 0.1-96 hr is preferable, and 0.5-48 hr is more preferable, and 2-24 hr is particularly preferable.

The pharmaceutical preparation comprising the adjuvant of the present invention can be applied to the intact skin or mucous membrane, but for the purpose of enhancing transdermal or transmucosal absorbability, it can also be applied to the skin or mucous membrane subjected to a physical or chemical treatment, such as skin abrading treatment or mucous membrane abrading treatment, treatment by microneedles, laser irradiation, thermal treatment, electric field treatment, magnetic field treatment, pressure treatment or alkali treatment. Furthermore, by means of a method using devices such as iontophoresis, electroporation, sonophoresis (ultrasonic wave), or by using a form of transdermal or transmucosal administration with a device equipped with microcannula, microneedles, needle-free injection, etc., an immune response with high safety against antigens can be established with even higher efficiency.

Among these, it is particularly preferable that transdermal or transmucosal administration is carried out by means of abrading, microneedles or needle-free injection. In addition, the above-described administration form is not particularly limited, and the most suitable administration means can be selected in accordance with permeability of the antigen in the skin or mucous membrane and necessary dosage.

In addition, another preferable method of administration of the pharmaceutical preparation is a method of administration using microneedles wherein a part or the entire surface of the needle part is coated with the pharmaceutical preparation comprising an antigen and the adjuvant of the present invention together with a base such as a carrier.

In addition, an antigen may be administered by microneedles in which a part or the entire surface of the needle part is coated with the antigen, while the pharmaceutical preparation comprising the adjuvant of the present invention may be applied to the skin or mucous membrane by its coating on or attaching to the skin or mucous membrane before or after antigen administration, or it may be applied to the skin or mucous membrane simultaneously with antigen administration.

For example, coating onto the needle part of microneedles is described in JP, A, 2004-504120, JP, A, 2004-528900, WO 2005/016440 and others.

One of the preferable methods of immunostimulation using the pharmaceutical preparation is a method in which an antigen is administered using microneedles by coating a part or the entire surface of the needle part of the microneedles with the antigen, and the pharmaceutical preparation comprising the adjuvant of the present invention is administered transdermally or transmucosally before or after the antigen administration, or simultaneously with the antigen administration. It is more preferable that, after administration of an antigen using microneedles by coating a part or the entire surface of the needle part of the microneedles with the antigen, the pharmaceutical preparation comprising the adjuvant of the present invention is attached.

Furthermore, among the methods in which an antigen is administered using microneedles by coating a part or the entire surface of the needle part of the microneedles with the antigen, and the pharmaceutical preparation is administered to the skin or mucous membrane simultaneously with the antigen administration or after the antigen administration, particular preference is given to the following method: administration of an antigen is carried out by puncture administration using microneedles, and the pharmaceutical preparation is applied (administered) over the entire microneedles used for the puncture on the skin or the mucous membrane; thus, co-administration of the pharmaceutical preparation comprising the adjuvant of the present invention and the antigen can be achieved in a single step.

In such a method, at the time when microneedles used for antigen administration are punctured on the skin or mucous membrane, the substrate surface without the punctured microneedles and the region of the skin or mucous membrane around said substrate surface which is not punctured by the microneedles are both covered by the pharmaceutical preparation, and fixed stably; then, simple and reliable administration can be realized.

The present invention is explained below in more detail by means of Examples, but the scope of the present invention is not limited to these Examples.

### (Example 1)

Dorsal hair of 8-week-old female hairless rats was shaved, and the rats were divided into (i) microneedle (OVA) application group, and (ii) microneedle (OVA) application + adjuvant-candidate application groups. The needle-tip part of microneedles was coated with 20 µg of OVA antigen, and the microneedles were puncture-administered for 30 min (the coating solution was an OVA/pullulan solution, and microneedles made of polylactic acid with a needle length of approximately 500 µm and 625 needles/cm² were used). Furthermore in the groups (ii), after puncture administration of the microneedles for 30 min, candidates of each adjuvant (glycerin, ethanol, and glycerin/ethanol (1/1) mixture solution) were transdermally administered to the puncture site for 6 hr. The application method of each adjuvant-candidate was as follows: regarding the glycerin, ethanol, and glycerin/ethanol (1/1) mixture solution, the pad part of a small-size tape for patch test (Torii Pharmaceutical Co., Ltd.) was impregnated with 120 µL of each of the adjuvant-candidate stock solution, then the tape was attached. In any group, fixation was carried out using Coban™ and SKINAGATE™.

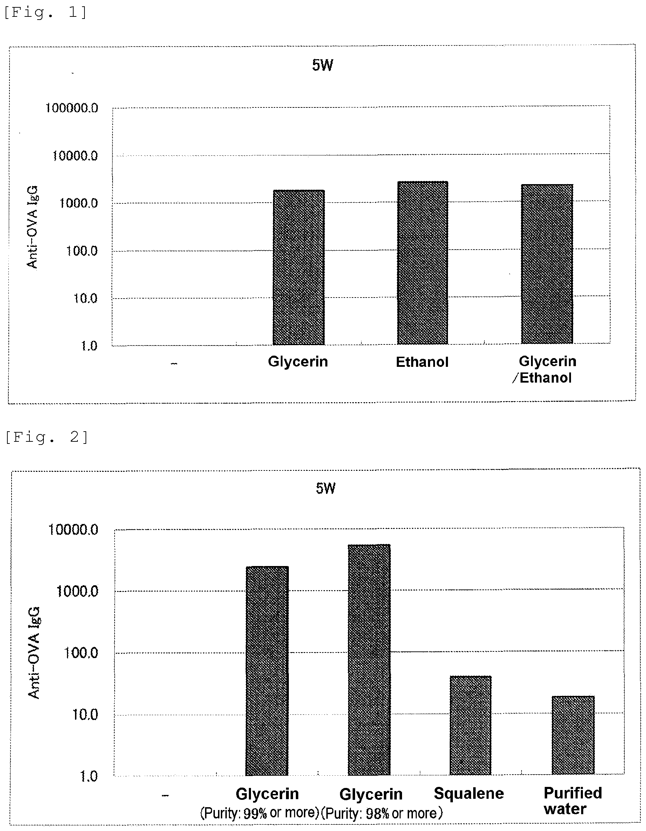

Administration of the antigen and adjuvant was carried out after 0, 2, and 4 weeks, the blood was collected after 2, 4, and 5 weeks, and OVA-specific IgG antibody titer was measured by ELISA (Fig. 1 shows antibody titers after 5 weeks).

Regarding the glycerin used in the Examples of the present specification, concentrated glycerin (Merck) was used in all the cases, except where otherwise stated.

As shown in Fig. 1, compared to the group in which OVA alone is administered via microneedles, all the adjuvant-candidate application groups exhibit significantly high IgG antibody titers, confirming that any of the glycerin, ethanol, and glycerin/ethanol 1/1 mixture solution has a high adjuvant effect.

### (Example 2)

In the above (ii) microneedle (OVA) application + adjuvant-candidate application groups, condition of the skin at 24 hr after detachment of each adjuvant was observed and the degree of skin irritation was evaluated with a score (Table 1).

As shown in Table 1, in the lauryl alcohol (tape) application group, while almost no irritation was observed immediately after detachment, some scales were observed in some of the rats at 24 hr after detachment. In addition, significant skin irritation accompanied by necrosis was observed in the ethanol application group, and similar skin irritation was also observed in the ethanol/glycerin mixture application group. Whereas in the glycerin application group, no skin irritation was observed, demonstrating that glycerin is an excellent adjuvant having a significant effect in increasing antibody titers without causing skin irritation.

Here, the above-mentioned tape preparation containing 40% lauryl alcohol (matrix-type tape preparation) was made as follows: 4.0 g of LA (NOF CORPORATION) and 13.3 g (6.0 g of dry weight) of DURO-TAK 87-2194 (acrylic adhesive from Henkel Corporation) were mixed and extended over a detachment liner, dried at 80°C for 15 min to make a thickness of 50 µm, to which a support was attached.

**[Table 1]**

| Skin irritation score. | |
|---|---|
| Adjuvant | Skin irritation score |
| Lauryl alcohol (tape) | -* |
| Glycerin (stock solution) | - |
| Ethanol (stock solution) | +++ |
| Glycerin (stock solution) / Ethanol (stock solution) = 1/1 | ++ |

| | |
|---|---|
| *(Some scales were observed at 24 hr after detachment) | |

### (Example 3)

Dorsal hair of 8-week-old female hairless rats was shaved, and the rats were divided into (i) microneedle (OVA) application group, and (ii) microneedle (OVA) application + adjuvant-candidate application groups. The needle-tip part of microneedles was coated with 20 µg of OVA antigen, and the microneedles were puncture-administered for 30 min (the coating solution was an OVA/pullulan solution, and microneedles made of polylactic acid with a needle length of approximately 500 µm and 625 needles/cm² were used). Furthermore in the groups (ii), after puncture administration of the microneedles for 30 min, candidates of adjuvants: glycerin (Merck), glycerin (Kao Chemicals), and squalene, and purified water as a control were used and the pad part of a small-size tape for patch test (Torii Pharmaceutical Co., Ltd.) was impregnated with 120 µL of each of these stock solutions, then the tape was attached to the puncture site for 6 hr. In any group, fixation was carried out using CobanTM and SKINAGATETM.

Administration of the antigen and adjuvant was carried out after 0, 2, and 4 weeks, the blood was collected after 2, 4, and 5 weeks, and OVA-specific IgG antibody titer was measured by ELISA (Fig. 2 shows antibody titers after 5 weeks).

As shown in Fig. 2, an adjuvant effect of the glycerin was confirmed even when manufacturers of the glycerin were changed. The glycerin used in the present invention is a concentrated glycerin (Merck: 99.0% or more of purity, Kao Chemicals: 98.0% or more of purity), and the adjuvant effect is not due to impurities, but is speculated to be due to the properties of glycerin itself.

Furthermore, squalene that shows no skin irritation similar to glycerin and has been previously used as an adjuvant in other companies showed a low IgG antibody titer similar to the control. From this, an excellent adjuvant effect of the glycerin was confirmed.

### (Example 4)

Dorsal hair of 8-week-old female hairless rats was shaved, and the rats were divided into (i) intradermic injection (OVA) application group, (ii) intradermic injection (OVA) application + glycerin application group, (iii) microneedle (OVA) application group, and (iv) microneedle (OVA) application + glycerin application group. Regarding (i) and (ii), OVA 20 µg/50 mL (saline) was intradermally administered. Regarding (iii) and (v), the needle-tip part of microneedles was coated with 20 µg of OVA antigen, and the microneedles were puncture-administered for 30 min (the coating solution was an OVA/pullulan solution, and microneedles made of polylactic acid with a needle length of approximately 500 µm and 625 needles/cm² were used). Furthermore, in the groups (ii) and (iv), after puncture administration of the microneedles for 30 min, a small-size tape for patch test (Torii Pharmaceutical Co., Ltd.), wherein its pad part was impregnated with 120 µL of glycerin, was attached to the puncture site for 6 hr. In any group, fixation was carried out using Coban™ and SKINAGATE™.

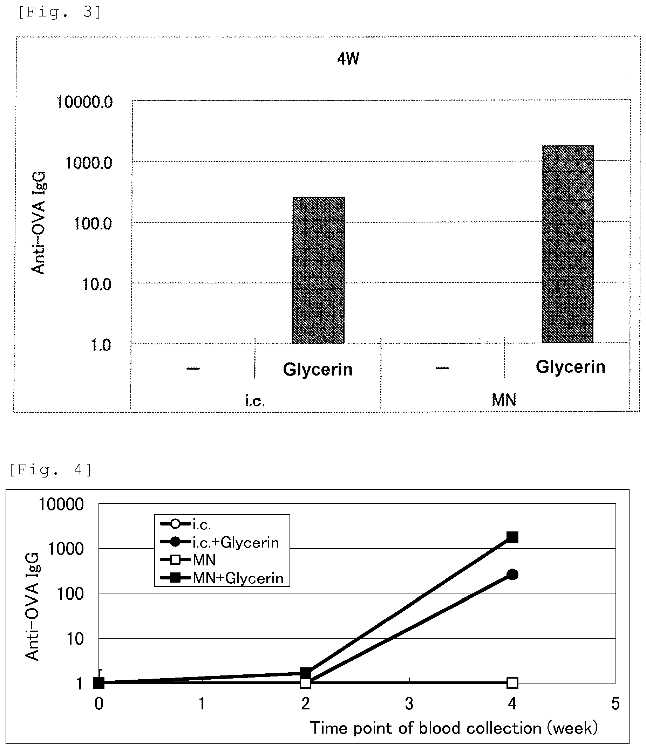

Administration of the antigen and adjuvant was carried out after 0 and 2 weeks, the blood was collected after 2 and 4 weeks, and OVA-specific IgG antibody titer was measured by ELISA (Fig. 3 shows antibody titers after 4 weeks, and Fig. 4 shows time-course changes in antibody titers).

As shown in Fig. 3, an adjuvant effect of the glycerin was exhibited more clearly by the microneedle application, compared to the intradermal injection application of OVA antigen. Moreover, as shown in Fig. 4, in the intradermal injection groups, no IgG antibody was detected after two weeks even when glycerin was co-used; whereas in the microneedle group with concomitant glycerin, an increase in the IgG antibody titer was observed from 2 weeks after.

Thus, it has been demonstrated that the adjuvant effect of glycerin can be obtained more clearly when the antigen is administered by microneedles, and that the IgG antibody titer raises at an earlier time point.

### (Example 5)

Dorsal hair of 8-week-old female hairless rats was shaved, and the rats were divided into (i) microneedle (OVA) application group, and (ii) microneedle (OVA) application + adjuvant-candidate (glycerin or glycerin derivative) application groups. The needle-tip part of microneedles was coated with 20 µg of OVA antigen, and the microneedles were puncture-administered for 30 min (the coating solution was an OVA/pullulan solution, and microneedles made of polylactic acid with a needle length of approximately 500 µm and 625 needles/cm² were used). Furthermore, in the groups (ii), after puncture administration of the microneedles for 30 min, as candidates of adjuvants, glycerin, glycerin derivative, as well as propylene glycol (PG), macrogol 400, monoglyceryl oleate (GMO) or triacetin as polyhydric alcohol were used, and the pad part of a small-size tape for patch test (Torii Pharmaceutical Co., Ltd.) was impregnated with 120 µL of each of these stock solutions, then the tape was attached to the puncture site for 6 hr. In any group, fixation was carried out using Coban™ and SKINAGATE™.

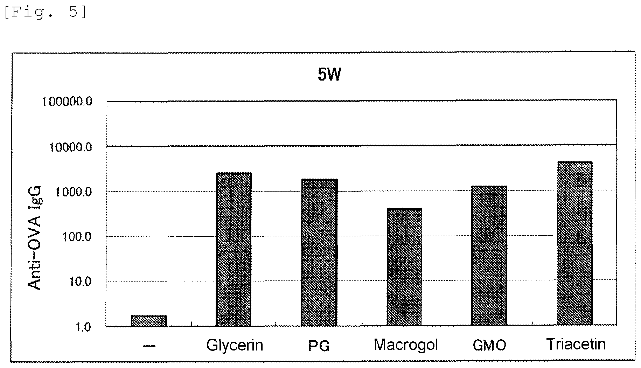

Administration of the antigen and adjuvant was carried out after 0, 2, and 4 weeks, the blood was collected after 2, 4, and 5 weeks, and OVA-specific IgG antibody titer was measured by ELISA (Fig. 5 shows antibody titers after 5 weeks).

As shown in Fig.5, an apparent effect to increase IgG antibody titer was observed in the groups wherein glycerin, glycerin derivative and polyacohol were administered as the adjuvant, compared to the group wherein OVA alone is administered. In particular, a strong adjuvant effect was exhibited by the glycerin with a carbon number of 3, PG and triacetin (all the three OH groups in the glycerin were acetylated).

Regarding the macrogol 400 as polyethylene glycols, and the GMO wherein along-chain hydrocarbon (C18) is ester-linked to one of the carbons in the glycerin, an adjuvant effect similar to that of low molecules such as glycerin was obtained. Therefore, it is considered that a higher adjuvant effect with better skin transition property can be obtained with glycerin, glycerin derivative and polyhydric alcohol.

Furthermore, not only glycerin, but also these glycerin derivatives do not cause skin irritation, demonstrating that they can be an adjuvant having high adjuvant effect and high safety.

### (Example 6)

Dorsal hair of 8-week-old female hairless rats was shaved, and the rats were subjected to microneedle (OVA) application + adjuvant (0, 25, or 75% glycerin solution) application. The needle-tip part of microneedles was coated with 20 µg of OVA antigen, and the microneedles were puncture-administered for 30 min (the coating solution was an OVA/pullulan solution, and microneedles made of polylactic acid with a needle length of approximately 500 µm and 625 needles/cm² were used). Thereafter, a small-size tape for patch test (Torii Pharmaceutical Co., Ltd.) wherein its pad part was impregnated with 120 µL of each of the adjuvant solutions was attached for 6 hr. In any group, fixation was carried out using Coban™ and SKINAGATE™.

Administration of the antigen and adjuvant was carried out after 0 and 2 weeks, the blood was collected after 2 and 4 weeks, and OVA-specific IgG antibody titer was measured by ELISA (Table 2 shows antibody titers after 4 weeks).

**[Table 2]**

| Glycerin concentration (%) | Adjuvant effect against anti-OVA IgG |
|---|---|
| 0 | none |
| 25 | none |
| 75 | present |

As shown in Table 2, an excellent adjuvant effect was confirmed for the glycerin concentration of 75 wt%. Here, a particularly excellent adjuvant effect can be obtained by glycerin concentration of 100 wt%.

### (Example 7)

Dorsal hair of 8-week-old female hairless rats was shaved, and the rats were divided into (i) microneedle (a mixture of OVA/pullulan/41 % glycerin) application group, and (ii) microneedle (OVA) application + adjuvant (100% glycerin) application group. The needle-tip part of microneedles was coated with 20 µg of OVA antigen, and the microneedles were puncture-administered for 30 min (the coating solution was an OVA/pullulan solution, and microneedles made of polylactic acid with a needle length of approximately 500 µm and 625 needles/cm² were used). Furthermore in the group (ii), after puncture administration of the microneedles for 30 min and being left stand for 10 min, a small-size tape for patch test (Torii Pharmaceutical Co., Ltd.) wherein its pad part was impregnated with 120 µL of the adjuvant solution was attached for 6 hr. In any group, fixation was carried out using Coban™ and SKINAGATE™.

Administration of the antigen and adjuvant was carried out after 0 and 2 weeks, the blood was collected after 2 and 4 weeks, and OVA-specific IgG antibody titer was measured by ELISA (Table 3 shows antibody titers after 4 weeks).

**[Table 3]**

| Administration form of adjuvant | Adjuvant effect against anti-OVA IgG |
|---|---|
| Simultaneously with antigen (mixture) | none |
| Glycerin was applied 10 min after antigen administration | present |

As shown in Table 3, no adjuvant effect was observed in the group in which a mixture of OVA and glycerin was administered via microneedles, whereas in the group in which glycerin was impregnation-attached after antigen administration, an apparent increase in the IgG antibody titer was observed. Furthermore, a high IgG antibody titer was also obtained when glycerin was impregnation-attached 10 min after administration of OVA.

It was also demonstrated that immune activity-enhancing effect can be obtained even when the adjuvant is attached after a certain time interval from antigen administration.

As described above, the adjuvant effect of glycerin of the present invention is shown to be unique not only in terms of effect and safety, but also in terms of administration, compared to other conventional adjuvants. Namely, it was demonstrated that the adjuvant effect of the present invention is not achieved by administration of a mixture with an antigen, as found in other adjuvants for injection purposes, but is achieved by applying the adjuvant separately from an antigen, in particular attaching or coating a high concentration of an adjuvant preparation after antigen is administered. Furthermore, it was clearly demonstrated that even when a high concentration of the adjuvant in an impregnated condition is attached, an increase in the antibody titer can be realized safely without skin irritation.

### [Industrial applicability]

As described above, the present invention provides a low-molecular adjuvant selected from glycerin and propylene glycol used for safe and efficient enhancement of immune activity of the skin. Namely, the adjuvant of the present invention is transdermally administered as it is, or is transdermally administered by coating after skin abrading treatment, or by using iontophoresis and microneedles, thereby realizing its wide utilization including evaluation of external pharmaceuticals, cosmetics or allergen substances, vaccine treatment of infectious disease, cancer, arteriosclerosis, cranial nerve disease such as Alzheimer's disease, and allergy, etc. In addition, it can be used as an anti-inflammatory immunomodulator for treating T cell-mediated diseases. Therefore, the present invention will make a great contribution to the development of pharmaceutical industry and related industries.

## Claims

1. An adjuvant comprising one or more selected from the group consisting of glycerin and propylene glycol that are polyhydric alcohols for use in the enhancement of immune activity when administered by transdermal or transmucosal application after antigen administration, wherein the adjuvant is contained at 85-100 wt% in a pharmaceutical preparation, wherein the use comprises administration of the antigen by subcutaneous injection or intradermal injection, transmucosal or transdermal administration of the antigen or administration of the antigen with microneedles before the adjuvant is applied.

2. Adjuvant according to Claim 1 for use according to Claim 1, wherein the pharmaceutical preparation is an ointment, a cream, a gel, a suppository, a hydrogel patch, a lotion, a solution, an impregnated-type preparation, or a blister.

3. Adjuvant according to Claim 1 or 2 for use according to Claim 1, wherein the adjuvant is contained at 95-100 wt% in the pharmaceutical preparation.

4. Adjuvant according to any one of Claims 1 to 3 for use according to Claim 1, wherein the pharmaceutical preparation is a matrix-type or laminated-type tape preparation or a reservoir-type preparation.

5. Adjuvant according to any one of Claims 1 to 4 for use according to Claim 1, which use comprises application of the pharmaceutical preparation to the intact skin or mucous membrane, or the skin or mucous membrane that had been subjected to physical or chemical treatment.

6. Adjuvant according to Claim 5 for use according to Claim 1, wherein the physical or chemical treatment is at least one of laser irradiation, skin abrading, or microneedle treatment, thermal treatment, ultrasonic treatment, electric field treatment, magnetic field treatment, pressure treatment or alkali treatment.

7. Adjuvant according to any one of Claims 1 to 6 for use according to Claim 1, which use comprises application of the pharmaceutical preparation by at least one of skin abrading, microneedles and needle-free injection.

8. Adjuvant according to any one of Claims 1 to 7 for use according to Claim 1, wherein a part or the entire surface of the needle part of the microneedles is coated with an antigen and/or an adjuvant.

9. Adjuvant according to any one of Claims 1 to 8 for use according to Claim 1, which use comprises application of the pharmaceutical preparation by at least one of lamellar structural changes, hydration, degeneration, small hole formation, peeling, or bypass formation in the stratum corneum.

10. Adjuvant according to Claim 9 for use according to Claim 1, which use comprises application of the pharmaceutical preparation by at least one of iontophoresis, sonophoresis, or electroporation.

11. Adjuvant according to any one of Claims 1 to 10 for use according to Claim 1, wherein the pharmaceutical preparation is comprised in a kit.

12. Adjuvant according to Claim 11 for use according to Claim 1, wherein the kit comprises an antigen or a vaccine, and an apparatus for antigen administration.

## Patentansprüche

1. Adjuvanz umfassend eines oder mehrere ausgewählt aus der Gruppe bestehend aus Glycerin und Propylenglycol, welche mehrwertige Alkohole sind, zur Verwendung bei der Verbesserung von Immunaktivität, wenn es durch transdermale oder transmukosale Applikation nach Antigen-Verabreichung verabreicht wird, wobei das Adjuvanz zu 85 bis 100 Gew.-% in einer pharmazeutischen Zubereitung enthalten ist, wobei die Verwendung eine Verabreichung des Antigens durch subkutane Injektion oder intradermale Injektion, eine transmukosale oder transdermale Verabreichung des Antigens oder eine Verabreichung des Antigens mit Mikronadeln umfasst, bevor das Adjuvanz appliziert wird.

2. Adjuvanz nach Anspruch 1 zur Verwendung nach Anspruch 1, wobei die pharmazeutische Zubereitung eine Salbe, eine Creme, ein Gel, ein Suppositorium, ein Hydrogelpflaster, eine Lotion, eine Lösung, eine Zubereitung vom Imprägniert-Typ oder ein Blister ist.

3. Adjuvanz nach Anspruch 1 oder 2 zur Verwendung nach Anspruch 1, wobei das Adjuvanz zu 95 bis 100 Gew.-% in der pharmazeutischen Zubereitung enthalten ist.

4. Adjuvanz nach einem der Ansprüche 1 bis 3 zur Verwendung nach Anspruch 1, wobei die pharmazeutischen Zubereitung eine Matrix-Typ- oder Laminiert-Typ-Klebeband-Zubereitung oder eine Reservoir-Typ-Zubereitung ist.

5. Adjuvanz nach einem der Ansprüche 1 bis 4 zur Verwendung nach Anspruch 1, wobei die Verwendung eine Applikation der pharmazeutischen Zubereitung auf die intakte Haut oder Schleimhaut oder die Haut oder Schleimhaut, die einer physikalischen oder chemischen Behandlung unterworfen worden war, umfasst.

6. Adjuvanz nach Anspruch 5 zur Verwendung nach Anspruch 1, wobei die physikalische oder chemische Behandlung zumindest eine Behandlung durch Laserbestrahlung, Hautabschabung oder eine Mikronadel, thermische Behandlung, Ultraschallbehandlung, Behandlung mit einem elektrischen Feld, Behandlung mit einem magnetischen Feld, Druckbehandlung oder Alkalibehandlung ist.

7. Adjuvanz nach einem der Ansprüche 1 bis 6 zur Verwendung nach Anspruch 1, wobei die Verwendung eine Applikation der pharmazeutischen Zubereitung durch zumindest eine Hautabschabung, Mikronadeln oder eine Nadel-freie Injektion umfasst.

8. Adjuvanz nach einem der Ansprüche 1 bis 7 zur Verwendung nach Anspruch 1, wobei ein Teil oder die gesamte Oberfläche des Nadelteils der Mikronadeln mit einem Antigen und/oder einem Adjuvanz beschichtet ist.

9. Adjuvanz nach einem der Ansprüche 1 bis 8 zur Verwendung nach Anspruch 1, wobei die Verwendung eine Applikation der pharmazeutischen Zubereitung durch zumindest lamellare Strukturveränderungen, Hydration, Degeneration, Kleinlochbildung, Schälen oder Bypassbildung im Stratum corneum umfasst.

10. Adjuvanz nach Anspruch 9 zur Verwendung nach Anspruch 1, wobei die Verwendung eine Applikation der pharmazeutischen Zubereitung durch zumindest eine lontophorese, Sonophorese oder Elektroporation umfasst.

11. Adjuvanz nach einem der Ansprüche 1 bis 10 zur Verwendung nach Anspruch 1, wobei die pharmazeutische Zubereitung in einem Kit enthalten ist.

12. Adjuvanz nach Anspruch 11 zur Verwendung nach Anspruch 1, wobei der Kit ein Antigen oder ein Vakzin und eine Vorrichtung zur Antigenverabreichung umfasst.

## Revendications

1. Adjuvant comprenant un ou plusieurs éléments sélectionnés parmi le groupe constitué de la glycérine et du propylèneglycol qui sont des alcools polyhydriques destiné à être utilisé dans l'amplification de l'activité immunitaire lors de l'administration par application transdermique ou transmuqueuse après administration d'antigène, dans lequel l'adjuvant est contenu à 85 à 100 % en poids dans une préparation pharmaceutique, dans lequel l'utilisation comprend l'administration de l'antigène par injection sous-cutanée ou injection intradermique, l'administration transmuqueuse ou transdermique de l'antigène ou l'administration de l'antigène avec des microaiguilles avant que l'adjuvant ne soit appliqué.

2. Adjuvant selon la revendication 1 destiné à être utilisé selon la revendication 1, dans lequel la préparation pharmaceutique est un onguent, une crème, un gel, un suppositoire, un patch hydrogel, une lotion, une solution, une préparation de type imprégnée ou un blister.

3. Adjuvant selon la revendication 1 ou 2 destiné à être utilisé selon la revendication 1, dans lequel l'adjuvant est contenu à 95 à 100 % en poids dans la préparation pharmaceutique.

4. Adjuvant selon l'une quelconque des revendications 1 à 3 destiné à être utilisé selon la revendication 1, dans lequel la préparation pharmaceutique est une préparation en ruban de type matrice ou de type stratifié ou une préparation de type réservoir.

5. Adjuvant selon l'une quelconque des revendications 1 à 4 destiné à être utilisé selon la revendication 1, laquelle utilisation comprend l'application de la préparation pharmaceutique à la peau ou membrane muqueuse intacte, ou la peau ou membrane muqueuse qui a été soumise à un traitement physique ou chimique.

6. Adjuvant selon la revendication 5 destiné à être utilisé selon la revendication 1, dans lequel le traitement physique ou chimique est au moins un parmi un traitement par irradiation laser, abrasion cutanée ou une microaiguille, un traitement thermique, un traitement ultrasonique, un traitement par champ électrique, un traitement par champ magnétique, un traitement par pression ou un traitement alcalin.

7. Adjuvant selon l'une quelconque des revendications 1 à 6 destiné à être utilisé selon la revendication 1, laquelle utilisation comprend l'application de la préparation pharmaceutique par au moins une abrasion cutanée, des microaiguilles ou une injection sans aiguille.

8. Adjuvant selon l'une quelconque des revendications 1 à 7 destiné à être utilisé selon la revendication 1, dans lequel une partie ou la surface entière de la partie d'aiguille des microaiguilles est enduite d'un antigène et/ou d'un adjuvant.

9. Adjuvant selon l'une quelconque des revendications 1 à 8 destiné à être utilisé selon la revendication 1, laquelle utilisation comprend l'application de la préparation pharmaceutique par au moins un procédé parmi des changements structurels lamellaires, l'hydratation, la dégénérescence, la formation de petits trous, le peeling ou la formation de pontage dans la couche cornée.

10. Adjuvant selon la revendication 9 destiné à être utilisé selon la revendication 1, laquelle utilisation comprend l'application de la préparation pharmaceutique par au moins un procédé parmi l'iontophorèse, la sonophorèse ou l'électroporation.

11. Adjuvant selon l'une quelconque des revendications 1 à 10 destiné à être utilisé selon la revendication 1, dans lequel la préparation pharmaceutique est comprise dans un kit.

12. Adjuvant selon la revendication 11 destiné à être utilisé selon la revendication 1, dans lequel le kit comprend un antigène ou un vaccin et un appareil pour l'administration d'antigène.
